# EUROPÄISCHE PATENTSCHRIFT

(11) **EP 0 453 060 B1**
(45) Veröffentlichungstag und Bekanntmachung des Hinweises auf die Patenterteilung: **23.07.1997**
(21) Anmeldenummer: 91250107.9
(22) Anmeldetag: 18.04.1991
(51) Int. Cl.: G01N 33/564, G01N 33/547

(54) **Verfahren zur Bestimmung von antinukleären Antikörpern**
Method for determination of antinuclear antibodies
Méthode pour déterminer des anti-corps anti-nucléaires

(30) Priorität: 19.04.1990 DD 339884
(43) Veröffentlichungstag der Anmeldung: 23.10.1991
(73) Patentinhaber: IMTEC IMMUNDIAGNOSTIKA GMBH, 16341 Zepernick (DE)
(72) Erfinder: Schössler, Werner, Dr., O-1297 Neu-Buch (DE); Pohl, Wolf-Detlef, Dr., O-1115 Berlin (DE); Butschak, Günter, Dr., O-1280 Bernau (DE); Dittrich, Christa, O-1115 Berlin (DE)

(56) Entgegenhaltungen:
- EP-A- 0 206 779
- EP-A- 0 335 293
- DD-A- 241 789
- DD-A- 262 672
- US-A- 3 997 657
- JOURNAL OF IMMUNOLOGICAL METHODS, vol. 35, 1980; R.M. HENDRY et al., Seiten 285-296/

## Beschreibung

Die Erfindung betrifft ein Verfahren zur qualitativen und/oder quantitativen Bestimmung von antinukleären Antikörpern. Das Verfahren findet Anwendung in der pharmazeutischen Industrie, den Biowissenschaften und in der Medizin.

Antinukleäre Antikörper (ANA, ANF) sind Autoantikörper unterschiedlicher Spezifität, die gegen Zellkernantigene gerichtet sind. Der Nachweis von ANA ist besonders bedeutsam für die Diagnose von Kollagenosen, so vor allem des systemischen Lupus erythematodes und der mit dieser Erkrankung eng assoziierten "mixed connective tissue disease (MCTD)", sowie von anderen Erkrankungen des rheumatischen Formenkreises.
Als Methode der Wahl zum Nachweis von antinukleären Antikörpern ist die indirekte Immunfluoreszenz gebräuchlich, die neben der Bestimmung des Titers eine Bewertung des Musters der Kernantikörper gestattet. Allerdings ist diese Methode mit den Nachteilen des hohen Zeitaufwandes, der hohen Kosten sowie der stark eingeschränkten Objektivierbarkeit behaftet.
Das Prinzip der indirekten Immunfluoreszenz beruht darauf, daß auf Objektträger fixierte Kryostatschnitte von Rattenleber (ca.5µm) oder epitheliale Zellinien (HEp-Zellen) mit verdünntem Patientenserum überschichtet werden, so daß sich vorhandene antinukleäre Antikörper an die nukleären Antikörper binden. Die so gebundenen Antikörper werden durch ein FITC-markiertes Antihuman-Immunglobulin nachgewiesen und mit einem geeigneten Fluoreszenzmikroskop sichtbar gemacht. Die Präparation der Kryostatschnitte von Rattenleber erfordert neben dem Aufwand der Tierhaltung eine kostenintensive Technik (Kryostat). Der Einsatz von Zellen ist an die kontinuierliche Züchtung derselben unter definierten und standardisierten Bedingungen gebunden und somit für die immunologische Routinediagnostik wenig geeignet. Dieser nicht unerhebliche Aufwand sowie die hiermit verbundenen Kosten limitieren die Zahl der Bestimmungen in der hochspezialisierten immunologischen Diagnostik, so daß diese den Anforderungen der Kliniken nicht mehr gerecht werden kann. Hinzu kommt, daß die Durchführung des Testes, die Beurteilung der Muster und die Interpretation der Befunde viel Erfahrung erfordern und somit dem Spezialisten vorbehalten sind. Besonders nachteilig sind die außerordentlich schwierige Standardisierbarkeit, die durch das zugrundeliegende Substrat sowie die eingesetzte Technik und Technologie determiniert wird, und die durch die visuelle Auswertung bedingte unzureichende Objektivierbarkeit.

Aus DD-A 262 672 ist ein Verfahren zur chemischen Aktivierung von festen Phasen bekannt, das sich zur Immobilisierung von biologischen Materialien eignet. Gemäß DD-A 262 672 werden mit Immunreagenzien beladene Träger durch nicht denaturierende Medien funktionell regeneriert.
Verfahren zur Bestimmung von antinukleären Antikörpern in Körperflüssigkeiten durch spezifische immunologische Bindung mit an einer festen Phase gebundenen Zellkernen sind in EP-A 0 335 293 und EP-A 0 206 779 beschrieben. Diese Verfahren haben jedoch den Nachteil, daß die angewendeten Vorbehandlungsmethoden zu dramatischen Veränderungen der Zellkerne führen und demzufolge nur Gemische verschiedener Zellkernfraktionen eingesetzt werden, die nur bedingt zur Erfassung von antinukleären Antikörpern in der Lage sind. So werden nach EP-A 0 335 293 die suspendierten Zellkerne mit 50 - 90%igem Aceton an der Unterlage fixiert. Die Folge ist eine Denaturierung der Organellen und eine weitgehende Elution der Lipide. Mit Zerstörung der Lipidmembran wird die Konformation der im Zellkern vorhandenen Antigene erheblich verändert. Gemäß EP-A 0 206 779 erfolgt eine Vorbehandlung der eingesetzten Zellsuspension mit Glutaraldehyd, Ultraschall und nachfolgende Mischung mit Triton X. Durch das als homobifunktionelles Reagenz bekannte Glutaraldehyd werden Oberflächenproteine quervernetzt und verändern ihre Antigenität. Die Ultraschallbehandlung führt zur Heraus lösung eines großen Teils der Antigene, was insbesondere auf die verwendeten Elektrolytlösungen zurückzuführen ist. Auch die nachfolgende Mischung mit Triton X wirkt weiter in Richtung Zellkernzerstörung, denn Triton X - der Handelsname außerordentlich oberflächenaktiver Verbindungen - wird bekanntermaßen zur Zerstörung von Membranen aus Zellen und Organellen (so auch Zellkernen) und zur nachfolgenden Isolierung der zunächst präparativ nicht zugänglichen Proteine, Nukleinsäuren etc. eingesetzt.

Es stellt sich daher die Aufgabe, isolierte Zellkerne so an eine feste Phase zu fixieren, daß sie ihre Struktur nicht verändern, und auf dieser Basis ein effektives Verfahren zur Bestimmung antinukleärer Antikörper im Immunoassay bereitzustellen.

Die Aufgabe konnte überrraschend dadurch gelöst werden, daß komplette, intakte Zellkerne unterschiedlicher Herkunft durch Dichtegradientenzentrifugation gewonnen und adsorptiv oder an eine zuvor chemisch aktivierte feste Phase gebunden werden. Die so immobilisierten intakten Zellkerne werden mit den zu untersuchenden Proben zur Bindung darin enthaltener antinukleärer Antikörper an die immobilisierten Zellkerne in geeigneten Puffern, die nichtionische oder ionische Detergenzien oder Proteine enthalten, in Kontakt gebracht, und die Bestimmung der gebundenen antinukleären Antikörper erfolgt im (Enzym-) Immunoassay. Gegebenenfalls werden die immobilisierten Zellkerne anschließend durch nicht denaturierende Medien funktionell regeneriert.

Hierzu wird die zu bestimmende Körperflüssigkeit, vorzugsweise Serum, in einer geeigneten Verdünnung mit den Zellkernen inkubiert, so daß sich vorhandene antinukleäre Antikörper an die Kernantigene der Zellkerne binden. Die so gebundenen Antikörper werden nunmehr mit einem markierten Anti-human-Antikörper nachgewiesen, wobei vorzugsweise Enzyme zur Markierung eingesetzt werden. Zellkerne aus Rattenleber kann man besonders vorteilhaft einsetzen, da diese die für die antinukleären Antikörper komplementären Kernantigene in hoher Konzentration und Dichte enthalten. Die Isolierung der Kernantigene erfolgt durch Dichtegradientenzentrifugation, so daß die antigenen Strukturen erhalten bleiben. Von großer Bedeutung ist, daß die so gewonnenen Zellkerne stabil und in hoher Dichte an die feste Phase, vorzugsweise Polystyren, gebunden werden, ohne daß sie ihre Struktur verändern. Der Zugang der antinukleären Antikörper ist dadurch sterisch kaum behindert. Dies ist vor allem durch die adsorptive oder kovalente Bindung der Kernantigene an zuvor chemisch aktiviertes Polystyren gegeben.
Der als feste Phase dienende Träger kann unterschiedliche geometrische Formen aufweisen, wobei die bekannten Mikrotitrationsplatten besonders geeignet sind. Einsetzbar sind auch Röhrchen, kugelförmige oder flächenförmige Träger.Die Bindung der antinukleären Antikörper erfolgt durch Inkubation des Serums in einer geeigneten Verdünnung mit den immobilisierten Zellkernen. Durch unterschiedliche Verdünnung des zu bestimmenden Serums ist eine Ermittlung des Titers und somit eine semiquantitative Bestimmung möglich. Eine quantitative Bestimmung ist bei Vorhandensein eines Standards prinzipiell möglich. Zur Unterdrückung unspezifischer Bindungen gestaltet sich der Einsatz von Puffern, die nichtionische oder ionische Detergentien,Inertproteine, wie Rinderserumalbumin, oder aber Serum anderer Spezies, wie Kälberserum, enthalten, als vorteilhaft.
Der Nachweis der gebundenen antinukleären Antikörper erfolgt durch spezifische enzymmarkierte Konjugate, die gegen humanes IgG, IgM oder IgA gerichtet sind. Für screening-Untersuchungen ist die gleichzeitige Erfassung aller Immunglobulinklassen vorzuziehen. Als Enzyme kommen vorzugsweise die Peroxidase und alkalische Phosphatase in Betracht.
Besonders vorteilhaft und kostengünstig gestaltet sich das Verfahren durch Abspaltung der gebundenen antinukleären Antikörper (und somit auch des Konjugates) mit nicht denaturierenden Medien, so daß die an der festen Phase (Polystyren) gebundenen Zellkerne funktionell regeneriert werden und erneut im Immunoassay eingesetzt werden können.
Als nicht denaturierende Medien sind Lösungen mit hohen Ionenstärken, chaotropischen Agenzien, depolarisierende Reagenzien, elektrostatische und Wasserstoffbrückenbindungen beeinflussende Substanzen sowie Haptene oder aber Puffer mit hohen bzw. niedrigen pH-Werten einsetzbar.
Die Vorteile des erfindungsgemäßen Verfahrens liegen vor allem in der effektiven Bestimmung großer Probenzahlen unter standardisierten Bedingungen, so daß den ständig wachsenden Anforderungen der klinischen Praxis entsprochen werden kann. Verbunden hiermit sind die wesentlich geringeren Kosten und der deutlich reduzierte Zeitaufwand des Verfahrens durch die objektiven Meßergebnisse und der damit verknüpften Möglichkeit der quantitativen Bestimmung der antinukleären Antikörper aus.
Von großer diagnostischer Relevanz ist die Differenzierbarkeit der ANA in unterschiedliche Immunglobulinklassen.

Die Erfindung wird im folgenden an Beispielen näher erläutert.

### Beispiel 1

Die von jungen Ratten gewonnene Rattenleber wird in physiologischer Kochsalzlösung abgekühlt und mit einer Schere zerkleinert. Anschließend wird sie mit einem Glas-Teflon-Homogenisator nach Potter bei etwa 2000 Umdrehungen pro Minute mit 8 - 10 Hüben in dem zehnfachen Volumen eines 0,01 M Phosphat-Puffers, pH 7,4, der 0,005 M MgCl₂ und 0,25 M Saccharose enthält (Puffer A), homogenisiert. Nachfolgend wird mit dem Homogenat ein 0,01 M Phosphat-Puffer, pH 7,4, der 0,005 M MgCl₂ und 0,34 M Saccharose enthält (Puffer B), überschichtet und schließlich in einer Kühlzentrifuge 5 min bei 1000 g und 4°C zentrifugiert. Der Niederschlag wird im Ausgangsvolumen des Puffers A, der zusätzlich 1% Triton enthält, aufgenommen und nochmals zentrifugiert. Der Waschschritt wird zweimal wiederholt.
Die Zellkerne werden in 2 ml eiskaltem 0,01 M Phosphat-Puffer aufgenommen und stehen für den Nachweis der antinukleären Antikörper zur Verfügung.

### Beispiel 2

Die entsprechend Beispiel 1 isolierten Zellkerne werden mit einem 0,1 M Phosphat-Puffer, pH 8,0, auf eine Konzentration von ca.500 eingestellt und in dieser Konzentration an chemisch aktivierte Polystyren-Mikrotestplatten über Nacht bei 4°C gebunden. Nach Entfernung der ungebundenen Zellkerne durch dreimaliges Waschen mit einem PBS-Puffer, pH 7,4, der 0,05% Tween 20 (Waschpuffer) enthält, werden eventuell noch reaktive Gruppen mit PBS-Puffer, pH 7,4, der 5% Kälberserum enthält, durch zweistündige Inkubation bei 37°C geblockt. Nach erneutem Waschen mit o. g. Waschpuffer werden die zu bestimmenden Seren 1 : 50 in einem PBS-Puffer, pH 7,4, der 0,1% Tween 20 und 5% Kälberserum enthält, verdünnt und 2 h bei 37°C mit den immobilisierten Zellkernen inkubiert. Nach erneutem dreimaligen Waschen mit genanntem Waschpuffer werden die so gebundenen antinukleären Antikörper mit einem antihuman-IgG-Peroxidase-Konjugat und nachfolgender Bestimmung mit H₂O₂ und o-Phenylendiamin und abschließender photometrischer Messung bei 492 nm nachgewiesen.

### Beispiel 3

Nach Beispiel 1 isolierte Zellkerne werden entsprechend Beispiel 2 an chemisch aktivierte Polystyren-Röhrchen gebunden, mit dem in Beispiel 2 angegebenen Puffer geblockt und anschließend mit den zu untersuchenden Seren in einer Verdünnung 1 : 50 in angegebenem Puffer inkubiert. Die so gebundenen antinukleären Antikörper werden nunmehr parallel mit einem anti-human-IgG-Peroxidase-Konjugat, anti-human-IgM-Peroxidase-Konjugat und anti-human-IgA-Peroxidase-Konjugat und nachfolgender Bestimmung der Enzymaktivität entsprechend Beispiel 2 nachgewiesen. Hierdurch werden antinukleäre Antikörper unterschiedlicher Immunglobulinklassen erfaßt.

### Beispiel 4

Nach Beispiel 1 isolierte Zellkerne werden entsprechend Beispiel 2 an chemisch aktivierte Mikrotitrationsplatten gebunden und anschließend - wie in Beispiel 2 und 3 angegeben - geblockt und mit den zu bestimmenden Seren inkubiert. Der Nachweis der antinukleären Antikörper erfolgt mit einem trivalenten Konjugat, das humanes IgG, IgM und IgA erfaßt, und Bestimmung der Enzymaktivität entsprechend Beispiel 2. Dieses Verfahren ist vor allem für screening-Untersuchungen empfehlenswert.

### Beispiel 5

Im Anschluß an die Durchführung des Immunoassays entsprechend den Beispielen 2 - 4 werden die Polystyren-Träger mit 1 M Kaliumthiocyanatlösung für 1 h bei Raumtemperatur inkubiert und somit die kovalent gebundenen Zellkerne funktionell regeneriert, so daß sie für einen erneuten Einsatz entsprechend den Beispielen 2 - 4 zur Verfügung stehen.

## Patentansprüche

1. Verfahren zur Bestimmung von antinukleären Antikörpern in Körperflüssigkeiten, gekennzeichnet dadurch,
daß komplette, intakte Zellkerne unterschiedlicher Herkunft durch Dichtegradientenzentrifugation gewonnen werden,
diese adsorptiv oder an eine zuvor chemisch aktivierte feste Phase, vorzugsweise an Polystyren, gebunden werden,
mit den zu untersuchenden Proben zur Bindung darin enthaltener antinukleärer Antikörper an die immobilisierten Zellkerne in geeigneten Puffern, die nichtionische oder ionische Detergenzien oder Proteine enthalten, in Kontakt gebracht werden,
nachfolgend eine Bestimmung der gebundenen antinukleären Antikörper im Immunoassay erfolgt,
und die immobilisierten Zellkerne ggf. anschließend durch nicht denaturierende Medien funktionell regeneriert werden.

2. Verfahren zur Bestimmung von antinukleären Antikörpern nach Anspruch 1, gekennzeichnet dadurch, daß die feste Phase eine unterschiedliche geometrische Form hat.

3. Verfahren zur Bestimmung von antinukleären Antikörpern nach Anspruch 1 - 2, gekennzeichnet dadurch, daß die als feste Phase dienenden Träger in Form einer Mikrotitrationsplatte oder eines Röhrchens vorliegen oder aber eine kugelförmige oder flächenförmige Gestalt haben.

4. Verfahren zur Bestimmung von antinukleären Antikörpern nach Anspruch 1 - 3, gekennzeichnet dadurch, daß die antinukleären Antikörper durch spezifische enzymmarkierte Konjugate, die gegen einzelne Immunglobulinklassen oder aber gegen mehrere gerichtet sind, nachgewiesen werden.

5. Verfahren zur Bestimmung von antinukleären Antikörpern nach Anspruch 1 - 4, gekennzeichnet dadurch, daß die nicht denaturierenden Medien Lösungen mit hohen Ionenstärken, chaotrophischen Agenzien, mit depolarisierenden Reagenzien, elektrostatische und Wasserstoffbrückenbindungenbeeinflussende Substanzen sowie mit Haptenen oder hohen bzw. niedrigen pH-Werten sind.

## Revendications

1. Méthode pour déterminer des anti-corps anti-nucléaires dans des liquides du corps, caractérisée en ce que
des noyaux intacts complets d'origine différente sont obtenus par centrifugation à gradient de densité,
que ceux-ci sont fixés par adsorption ou à une phase solide, préalablement activée par voie chimique, de préférence au polystyrène,
qu'ils sont mis en contact avec les prélèvements à examiner dans le but de fixer les anti-corps antinucléaires y existants aux noyaux immobilisés dans des tampons appropriés contenant des détergents non ioniques ou ioniques ou des protéines, que la détermination des anti-corps antinucléaires fixés se fait ensuite dans l'immunoassay
et que, le cas échéant, les noyaux immobilisés sont ensuite l'objet d'une régénération fonctionnelle par des milieux non dénaturants.

2. Méthode pour déterminer des anti-corps antinucléaires selon la revendication 1, caractérisée en ce que la phase solide a une forme géométrique différente.

3. Méthode pour déterminer des anti-corps antinucléaires selon les revendications 1 et 2, caractérisés en ce que les supports servant de phase solide soit constituent une plaque de microtitration ou une tube soit se présentent sous forme sphérique ou de surface plane.

4. Méthode pour déterminer des anti-corps antinucléaires selon les revendications 1 à 3, caractérisée en ce que les anti-corps nucléaires sont décelés par des liaisons conjuguées spécifiques et marquées par enzymes, lesquelles sont dirigées contre des classes individuelles ou contre plusieurs classes d'immunoglobuline.

5. Méthode pour déterminer des anti-corps antinucléaires selon les revendications 1 à 4, caractérisée en ce que les milieux non dénaturants représentent des solutions à force ionique élevée, aux agents chaotrophiques, aux réactifs dépolarisants, aux substances avec influence sur les liaisons électrostatiques et hydrogènes ainsi qu'aux haptènes ou aux valeurs pH élevées ou faibles.

## Claims

1. A method for determination of antinuclear antibodies in body fluids characterized by the fact
that complete, intact cell nuclei of various origin are obtained by density gradient centrifugation,
which are bound by adsorption or a solid phase previously chemically activated, preferently polystyrene,
brought into contact with the specimens to be analyzed for binding the antibodies contained therein to the immobilized cell nuclei in appropriate buffers containing non-ionic or ionic detergents or proteins,
subsequently the bound antinuclear antibodies are determined in an immunoassay, and the immobilized cell nuclei are, if possible, subsequently functionally regenerated by non-denaturing media.

2. A method for determination of antinuclear antibodies according to claim 1 characterized by the fact that the solid phase has a variable geometric form.

3. A method for determination of antinuclear antibodies according to claims 1 - 2 characterized by the fact that the carriers serving as solid phase are available in the form of a microtitration plate or a tube or have a spheric or planiform shape.

4. A method for determination of antinuclear antibodies according to claims 1-3 characterized by the fact that the antinuclear antibodies are detected by specific enzyme marked conjugates directed against single or several immunoglobulin classes.

5. A method for determination of antinuclear antibodies according to claims 1 - 4, characterized by the fact that the non-denaturing media are solutions with a high ionic strength, chaotrophic agents, with depolarizing reagents, electrostatic substances and substances affecting hydrogen bridge bond compounds as well as with haptens or high or low pH-values.
